# EUROPÄISCHE PATENTSCHRIFT

(11) **EP 4 297 699 B1**
(45) Veröffentlichungstag und Bekanntmachung des Hinweises auf die Patenterteilung: **13.08.2025**
(21) Anmeldenummer: 22712303.1
(22) Anmeldetag: 23.02.2022
(51) Int. Cl.: A61F 2/30, A61F 2/38

(54) **KNOCHENIMPLANTATSYSTEM, KNOCHENIMPLANTAT UND VERFAHREN ZUR HERSTELLUNG EINES SOLCHEN KNOCHENIMPLANTATS**
BONE IMPLANT SYSTEM, BONE IMPLANT AND METHOD OF MANUFACTURING SUCH A BONE IMPLANT
SYSTÈME D'IMPLANT OSSEUX, IMPLANT OSSEUX ET PROCÉDÉ DE FABRICATION D'UN TEL IMPLANT OSSEUX

(30) Priorität: 25.02.2021 DE 102021201783
(43) Veröffentlichungstag der Anmeldung: 03.01.2024
(73) Patentinhaber: Aesculap AG, 78532 Tuttlingen (DE)
(72) Erfinder: HETTICH, Georg, 79117 Freiburg (DE)
(74) Vertreter: Patentanwälte Ruff, Wilhelm, Beier, Dauster & Partner mbB
(86) Internationale Anmeldenummer: PCT/EP2022/054544
(87) Internationale Veröffentlichungsnummer: WO 2022/180107

(56) Entgegenhaltungen:
- EP-A2- 0 517 030
- EP-B1- 0 093 869
- DE-B4- 10 308 141
- US-A1- 2002 029 084

## Beschreibung

Die Erfindung betrifft ein Knochenimplantatsystem, insbesondere zum Auffüllen von Knochendefekten und/oder zum Stabilisieren eines Gelenkersatzimplantats. Die Erfindung betrifft zudem ein unter Verwendung eines solchen Knochenimplantatsystems hergestelltes Knochenimplantat und ein Verfahren zur Herstellung eines solchen Knochenimplantats.

Knochenimplantate sind im Bereich der orthopädischen Chirurgie allgemein bekannt und finden beispielsweise bei Gelenkersatzoperationen im Knie-, Hüft-, Schulter- und/oder Sprunggelenksbereich Verwendung.

Bei Kniegelenkersatzoperationen werden vorhandene metaphysäre Knochendefekte in der proximalen Tibia oder dem distalen Femur üblicherweise mit einem Knochenimplantat behandelt. Das Knochenimplantat wird vor der Implantierung des eigentlichen tibialen oder femoralen Gelenkersatzimplantats implantiert und soll zum einen den Knochendefekt auffüllen. Zum anderen soll das Knochenimplantat das Gelenkersatzimplantat im implantierten Zustand zusätzlich stabilisieren. Entsprechende Knochenimplantate werden oftmals auch als Cone oder metaphysärer Cone bezeichnet.

Um eine erfolgreiche Behandlung zu gewährleisten, muss die Außenkontur des Cones auf eine Innenkontur des zu behandelnden Knochendefekts abgestimmt sein. Hierfür sind einstückig gefertigte Cones in unterschiedlichen Größen und Formen erhältlich. Dennoch ist nicht in jedem Fall eine optimale Anpassung an einen vorhandenen Knochendefekt möglich, da nicht für jede denkbare Defektgeometrie ein entsprechend angepasster Cone gefertigt und vorgehalten werden kann. Alternativ kommen vereinzelt individuell gefertigte einstückige Cones zum Einsatz. Deren Herstellung ist jedoch zeit- und kostenintensiv.

Die EP 0517030 A2 offenbart ein Knochenimplantatsystem, welches Scheibenelemente verschiedener Stärke und mit unterschiedlichen Außendurchmessern umfasst. Die Scheibenelemente dienen dazu, ein Wirbelkörperimplantat durch Stapelung der Scheibenelemente individuell zu konfigurieren. Hierzu wird zunächst der benötigte Außendurchmesser festgelegt und anschließend Scheibenelemente unterschiedlicher Stärke gestapelt, um die gewünschte Implantathöhe zu erhalten. Es ist keine Kombination von Scheibenelementen mit unterschiedlichen Durchmessern vorgesehen, weshalb die Außenkontur des Implantats im Gegensatz zur vorliegenden Erfindung nicht individualisierbar ist.

Aufgabe der Erfindung ist es, ein Knochenimplantatsystem, ein Knochenimplantat und ein Verfahren zur Herstellung eines solchen Knochenimplantats bereitzustellen, welche die eingangs im Zusammenhang mit konventionellen Knochenimplantaten genannten Nachteile teilweise oder vollständig vermeiden.

Diese Aufgabe wird gelöst durch ein Knochenimplantatsystem mit den Merkmalen des Anspruchs 1, ein Knochenimplantat mit den Merkmalen des Anspruchs 8 und ein Verfahren mit den Merkmalen des Anspruchs 9. Bevorzugte Ausgestaltungen sind Gegenstand der Unteransprüche sowie der Beschreibung.

Das erfindungsgemäße Knochenimplantatsystem weist mehrere unterschiedliche Scheibenelemente mit unterschiedlichen Außendurchmessern auf, wobei die Scheibenelemente unter Ausbildung unterschiedlicher Stapelanordnungen unterschiedlich kombiniert axial übereinanderstapelbar sind, wobei die unterschiedlichen Stapelanordnungen aufgrund der unterschiedlichen Außendurchmesser der Scheibenelemente verschiedene Außenkonturen aufweisen, und wobei die Scheibenelemente jeweils einen an einer Oberseite angeordneten Formschlussabschnitt und einen an einer axial gegenüberliegenden Unterseite angeordneten komplementären Formschlussabschnitt aufweisen, mittels derer die Scheibenelemente in den unterschiedlichen Stapelanordnungen in Radialrichtung und/oder Umfangsrichtung formschlüssig aneinander festlegbar sind. Durch die erfindungsgemäße Lösung kann auf ein zeit- und kostenintensives Herstellen individualisierter einstückiger Knochenimplantate verzichtet werden. Gleichzeitig kann dennoch eine gegenüber üblichen, nicht-individualisierten Knochenimplantaten verbesserte Anpassung des Knochenimplantats an eine gegebene Geometrie des zu behandelnden Knochendefekts erreicht werden. Vereinfacht ausgedrückt werden hierfür - je nach Geometrie des Knochendefekts - mehrere Scheibenelemente des Knochenimplantatsystems derart ausgewählt und kombiniert axial übereinandergestapelt, dass eine anforderungsgerechte Übereinstimmung der Außenkontur der resultierenden Stapelanordnung mit der Innenkontur des Knochendefekts erreicht wird. Durch die unterschiedlichen Außendurchmesser der Scheibenelemente und die Möglichkeit, die Scheibenelemente unterschiedlich kombiniert übereinanderzustapeln, lassen sich unterschiedliche Stapelanordnungen und dementsprechend unterschiedliche Außenkonturen bilden. Dabei dienen die Formschlussabschnitte und die komplementären Formschlussabschnitte einer formschlüssigen Verbindung der Scheibenelemente in der jeweils gebildeten Stapelanordnung. Vereinfacht ausgedrückt sind die Formschlussabschnitte der unterschiedlichen Scheibenelemente jeweils vorzugsweise gleich, gleichartig, übereinstimmend, identisch und/oder gegeneinander austauschbar gestaltet. In anderen Worten ausgedrückt ist die Gestaltung der Formschlussabschnitte vorzugsweise unabhängig von dem Außendurchmesser des jeweiligen Scheibenelements. Entsprechendes gilt vorzugsweise sinngemäß im Hinblick auf die komplementären Formschlussabschnitte. Dies gewährleistet, dass die Scheibenelemente - unabhängig von der jeweils gewählten Kombination und/oder gebildeten Stapelanordnung - stets aneinander festlegbar sind. Die mehreren Scheibenelemente können beispielsweise ein erstes Scheibenelement mit einem ersten Außendurchmesser, ein zweites Scheibenelement mit einem zweiten Außendurchmesser und ein drittes Scheibenelement mit einem dritten Außendurchmesser umfassen. Die Außendurchmesser sind in Bezug auf ihr Durchmessermaß und/oder ihre Formgebung unterschiedlich. Insoweit können die Scheibenelemente, genauer: deren Außendurchmesser, insbesondere kreis- und/oder ellipsenförmig und/oder im weitesten Sinne abgerundet sein.

Selbstverständlich kann das Knochenimplantatsystem auch mehrere Scheibenelemente mit ein- und demselben Außendurchmesser aufweisen. Beispielsweise können mehrere erste Scheibenelemente, mehrere zweite Scheibenelemente und mehrere dritte Scheibenelemente vorgesehen sein. Die unterschiedlichen Scheibenelemente unterscheiden sich wenigstens im Hinblick auf ihren Außendurchmesser, insbesondere dessen Maß- und/oder Formgebung. Zusätzlich können unterschiedlich dicke Scheibenelemente vorgesehen sein, wobei diese nicht notwendigerweise gleichzeitig einen unterschiedlichen Außendurchmesser aufweisen müssen. Die Scheibenelemente sind vorzugsweise jeweils als Kreisscheibe mit kreisförmigem Querschnitt gestaltet. Vorzugsweise sind die unterschiedlichen Außendurchmesser graduell abgestuft dimensioniert. Hierdurch können insbesondere konische Außenkonturen mit unterschiedlichen Konuswinkeln ausgebildet werden. Die Formschlussabschnitte können jeweils insbesondere eine Verbindungsnut, einen Verbindugnsstift, eine Verzahnung oder dergleichen zum formschlüssigen Zusammenwirken mit jeweils einem der komplementären Formschlussabschnitte aufweisen. Dementsprechend können die komplementären Formschlussabschnitte jeweils insbesondere eine Passfeder, eine Aufnahmebohrung, eine Gegenverzahnung oder dergleichen aufweisen. Die Formschlussabschnitte und/oder die komplementären Formschlussabschnitte können einstückig mit dem jeweiligen Scheibenelement ausgebildet sein. Alternativ können die Formschlussabschnitte und/oder die komplementären Formschlussabschnitte separat gefertigt und hiernach mit dem jeweiligen Scheibenelement zusammengefügt sein.

In Ausgestaltung der Erfindung sind die Scheibenelemente jeweils ringförmig gestaltet und weisen einen, vorzugsweise identischen, Innendurchmesser auf. In axial übereinandergestapeltem Zustand bilden die Innendurchmesser der Scheibenelemente eine, vorzugsweise kreiszylindrische, Aufnahmeaussparung. Die Aufnahmeaussparung ist zur Aufnahme eines Schaftabschnitts eines Gelenkersatzimplantats vorgesehen. Vorzugsweise ist eine kreisförmige und/oder eine elliptische Ringform vorgesehen.

In weiterer Ausgestaltung der Erfindung weisen die Scheibenelemente jeweils wenigstens einen in Axialrichtung von der Oberseite oder der Unterseite aufragenden Abstandsabschnitt auf, wodurch die Scheibenelemente in den unterschiedlichen Stapelanordnungen unter Ausbildung von Radialspalten übereinanderstapelbar sind. Sofern das aus dem Knochenimplantatsystem gebildete Knochenimplantat unter der Verwendung einer Verbindungsmasse, wie beispielsweise Knochenzement, in dem Knochendefekt verankert wird, erlauben die Radialspalte ein Eindringen der Verbindungsmasse zwischen die einzelnen Scheibenelemente. Hierdurch kann die aus den Scheibenelementen gebildete Stapelanordnung bzw. das Knochenimplantat zusätzlich stabilisiert werden. Mit anderen Worten können die Scheibenelemente aufgrund der vorhandenen Abstandsabschnitte "auf Lücke" gestapelt werden. Die Verbindungsmasse kann dann durch diese Lücken zwischen die einzelnen Scheibenelemente gelangen. Die Abstandsabschnitte können einstückig an dem jeweiligen Scheibenelement ausgebildet sein. Alternativ können die Abstandsabschnitte jeweils als separates Bauteil gefertigt und hiernach mit dem jeweiligen Scheibenelement zusammengefügt sein.

In weiterer Ausgestaltung der Erfindung weisen die Scheibenelemente jeweils wenigstens eine in die Oberseite oder die Unterseite eingesenkte Aufnahmeaussparung auf, die zur Aufnahme einer Verbindungsmasse vorgesehen ist. Dies ist insbesondere in Verbindung mit den Merkmalen der vorhergehenden Ausgestaltung besonders vorteilhaft. Sofern Radialspalte vorgesehen sind, kann die Verbindungsmasse, beispielsweise Knochenzement, durch die Radialspalte radial zwischen die Scheibenelemente eindringen und in den Aufnahmeaussparungen aufgenommen werden. Sofern keine Radialspalte vorhanden sind, können die Aufnahmeaussparungen vor dem Übereinanderstapeln der Scheibenelemente mit der Verbindungsmasse befüllt werden. Hierdurch kann die gebildete Stapelanordnung/das Knochenimplantat in sich fixiert werden. Die Aufnahmeaussparungen können auch als Aufnahmetaschen bezeichnet werden. Die Aufnahmeaussparungen bilden vorzugsweise keine durchgängige Verbindung zwischen der Ober- und Unterseite des jeweiligen Scheibenelements. Dies im Unterschied zu einer einfachen Axialbohrung.

In weiterer Ausgestaltung der Erfindung weisen die Formschlussabschnitte jeweils wenigstens einen axial aufragenden Verbindungsabschnitt auf, und die komplementären Formschlussabschnitte weisen jeweils wenigstens eine in Axialrichtung eingebrachte Aufnahmebohrung auf oder umgekehrt. Die Verbindungsstifte ragen vorzugsweise von den Oberseiten der Scheibenelemente ab. Die Aufnahmebohrungen sind vorzugsweise in die Unterseiten der Scheibenelemente eingebracht. Alternativ kann eine hierzu umgekehrte Anordnung vorgesehen sein. Die Verbindungsstifte und die Aufnahmebohrungen sind maßlich aufeinander abgestimmt. In formschlüssig aneinander festgelegtem Zustand der Scheibenelemente greifen die Verbindungsstifte in die Aufnahmebohrungen ein, so dass die jeweils gebildete Stapelanordnung in Radial- und/oder Umfangsrichtung in sich fixiert ist. Im Vergleich zu weiteren denkbaren Gestaltungen der Formschlussabschnitte und/oder der komplementären Formschlussabschnitte erlaubt diese Ausgestaltung der Erfindung eine einfache Herstellung und hohe Beanspruchbarkeit der formschlüssigen Verbindung. Vorzugsweise sind die Formschlussabschnitte und die komplementären Formschlussabschnitte derart aufeinander abgestimmt, dass die Scheibenelemente in unterschiedlich relativ zueinander rotierten Positionen aneinander festlegbar sind. Dies wird bevorzugt dadurch erreicht, dass eine Anzahl der Aufnahmebohrungen größer ist als eine Anzahl der Verbindungsstifte. Vorzugsweise sind die Aufnahmebohrungen und/oder die Verbindungsstifte in Radialrichtung konzentrisch angeordnet.

In weiterer Ausgestaltung der Erfindung weisen die Scheibenelemente jeweils mehrere in Umfangsrichtung versetzt angeordnete Verbindungsstifte und/oder Aufnahmebohrungen auf, wobei die Anordnungen der Verbindungsstifte und/oder der Aufnahmebohrungen der unterschiedlichen Scheibenelemente ein identisches Bohrbild ausbilden. Hierdurch wird vereinfacht ausgedrückt ermöglicht, dass sämtliche Scheibenelemente untereinander formschlüssig verbindbar sind. Das Bohrbild der Verbindungsstifte kann charakterisiert werden durch den Durchmesser der Verbindungsstifte, den Durchmesser eines Lochkreises, auf welchen die mehreren Verbindungsstifte des jeweiligen Scheibenelements angeordnet sind, eine Anzahl der Verbindungsstifte je Lochkreis und/oder eine Winkelstellung der Verbindungsstifte auf dem Lochkreis. Entsprechendes gilt sinngemäß im Hinblick auf das Bohrbild der Aufnahmebohrungen. Vorzugsweise sind jeweils wenigstens zwei Verbindungsstifte und/oder Aufnahmebohrungen vorgesehen und um 180° zueinander versetzt auf dem jeweiligen Lochkreis angeordnet. Selbstverständlich könnten auch mehr als zwei Verbindungsstifte und/oder Aufnahmebohrungen vorgesehen sein. So beispielsweise drei, vier, fünf, sechs oder mehr Verbindungsstifte und/oder Aufnahmebohrungen. Diese sind vorzugsweise jeweils um 120°, 90°, 72° bzw. 60° zueinander in Umfangsrichtung versetzt angeordnet.

In weiterer Ausgestaltung der Erfindung sind wenigstens drei, bevorzugt mehr als sechs, besonders bevorzugt mehr als zwölf unterschiedliche Scheibenelemente vorgesehen. Diese weisen jeweils einen unterschiedlichen Außendurchmesser auf. Dabei können mehrere Scheibenelemente mit ein- und demselben Außendurchmesser vorgesehen sein, so dass das Knochenimplantatsystem dementsprechend insgesamt mehr als die genannten wenigstens drei, bevorzugt mehr als sechs, besonders bevorzugt mehr als zwölf, unterschiedlichen Scheibenelemente aufweist. Sind beispielsweise von jedem Außendurchmesser drei Scheibenelemente vorhanden, weist das Knochenimplantatsystem insgesamt wenigstens 9, bevorzugt mehr als 18, besonders bevorzugt mehr als 36, (unterschiedliche) Scheibenelemente auf.

Das erfindungsgemäßen Knochenimplantat weist mehrere in Axialrichtung übereinandergestapelte Scheibenelemente mit unterschiedlichen Außendurchmessern auf, wobei die übereinandergestapelten Scheibenelemente mittels oberseitig an den Scheibenelementen angeordneter Formschlussabschnitte und unterseitig an den Scheibenelementen angeordneter komplementärer Formschlussabschnitte in Radialrichtung und/oder Umfangsrichtung formschlüssig aneinander festgelegt sind. Die Außendurchmesser sind insbesondere im Hinblick auf ihre Maß- und/oder Formgebung unterschiedlich. Zu mit der erfindungsgemäßen Gestaltung des Knochenimplantats einhergehenden Vorteilen wird zur Vermeidung von Wiederholungen auf die Beschreibung zu dem erfindungsgemäßen Knochenimplantatsystem verwiesen. Das dort Gesagte gilt sinngemäß auch im Hinblick auf das erfindungsgemäße Knochenimplantat. Bevorzugte Ausgestaltungen des Knochenimplantats ergeben sich sinngemäß aus den Merkmalen der Ansprüche 2 bis 7.

Das erfindungsgemäße Verfahren zur Herstellung eines individualisierten Knochenimplantats unter Verwendung eines Knochenimplantatsystems nach der vorhergehenden Beschreibung weist die Schritte auf: Erfassen einer Innenkontur eines Knochendefekts; Ermitteln einer zum Auffüllen des Knochendefekts erforderlichen Außenkontur des Knochenimplantats in Abhängigkeit der erfassten Innenkontur; Auswählen, Stapeln und Aneinanderfestlegen mehrerer unterschiedlicher Scheibenelemente des Knochenimplantatsystems, wobei die mehreren Scheibenelemente in Abhängigkeit der ermittelten Außenkontur ausgewählt und/oder gestapelt werden. Vorzugsweise wird die Innenkontur des Knochendefekts intra-operativ erfasst. Die Innenkontur kann beispielsweise durch einen 3D-Datensatz repräsentiert werden. Das Erfassen erfolgt messtechnisch, beispielsweise mittels einer Time-of-Flight-Kamera. Die erforderliche Außenkontur des Knochenimplantats wird in Abhängigkeit der erfassten Innenkontur ermittelt. Das Ermitteln erfolgt vorzugsweise computerbasiert. Die zur Abbildung der erforderlichen Außenkontur notwendigen Scheibenelemente werden von medizinischem Personal, bevorzugt einem Operateur, aus der Gesamtmenge der vorhandenen unterschiedlichen Scheibenelemente des Knochenimplantatsystems ausgewählt. Auch dies erfolgt vorzugsweise computergestützt, beispielsweise mittels eines hierfür eingerichteten Optimierungsverfahrens. Das Auswählen umfasst zum einen die eigentliche Auswahl und zum anderen die zur Abbildung der erforderlichen Außenkontur erforderliche Kombination der Scheibenelemente in axialer Richtung. Hiernach werden die ausgewählten Scheibenelemente, vorzugsweise manuell, gestapelt und mittels der Formschlussabschnitte und komplementären Formschlussabschnitte in Radialrichtung und/oder Umfangsrichtung formschlüssig aneinander festgelegt. Hiernach kann das auf diese Weise hergestellte Knochenimplantat auf grundsätzlich bekannte Weise in den Knochendefekt eingesetzt und in demselben verankert werden. Das Auswählen der Scheibenelemente und deren Zusammenbau muss nicht zwingend intra-operativ erfolgen. Vielmehr kann die Innenkontur des Knochendefekts im Vorfeld einer auszuführenden Operation erfasst werden, wobei das Auswählen und Zusammensetzen der Scheibenelemente hierauf basierend im Vorfeld der Operation erfolgen kann.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt in schematisch stark vereinfachter Darstellung eine Ausführungsform eines erfindungsgemäßen Knochenimplantatsystems mit mehreren unterschiedlichen Scheibenelementen,
- Fig. 2, 3, 4: ein exemplarisches erstes Scheibenelement des Knochenimplantatsystems nach Fig. 1 in einer schematischen Draufsicht (Fig. 2), einer schematischen Schnittdarstellung entlang einer Schnittlinie III-III nach Fig. 2 (Fig. 3) und einer schematischen Untersicht (Fig. 4),
- Fig. 5, 6, 7: ein exemplarisches zweites Scheibenelement des Knochenimplantatsystems nach Fig. 1 in einer schematischen Draufsicht (Fig. 5), einer schematischen Schnittdarstellung entlang einer Schnittlinie VI-VI nach Fig. 5 (Fig. 6) und einer schematischen Untersicht (Fig. 7),
- Fig. 8, 9, 10: ein exemplarisches drittes Scheibenelement des Knochenimplantatsystems nach Fig. 1 in einer schematischen Draufsicht (Fig. 8), einer schematischen Schnittdarstellung entlang einer Schnittlinie IX-IX nach Fig. 8 (Fig. 9) und einer schematischen Untersicht (Fig. 10),
- Fig. 11: in einer im Vergleich zu den Fig. 3, 6 sowie 9 um 90° gedrehten Schnittansicht das erste, zweite und dritte Scheibenelement in einer axial übereinandergestapelten Stapelanordnung,
- Fig. 12: die Stapelanordnung nach Fig. 11, wobei die Scheibenelemente in Radialrichtung und/oder Umfangsrichtung formschlüssig aneinander festgelegt sind,
- Fig. 13: eine schematisch stark vereinfachte Darstellung einer Ausführungsform eines erfindungsgemäßen Knochenimplantats, das unter Verwendung des Knochenimplantatsystems nach Fig. 1 ausgebildet ist, wobei das Knochenimplantat zum Auffüllen eines Knochendefekts proximal in eine Tibia implantiert ist, und
- Fig. 14: ein schematisch stark vereinfachtes Flussdiagramm zur Verdeutlichung einer Ausführungsform eines erfindungsgemäßen Verfahrens zur Herstellung eines erfindungsgemäßen Knochenimplantats.

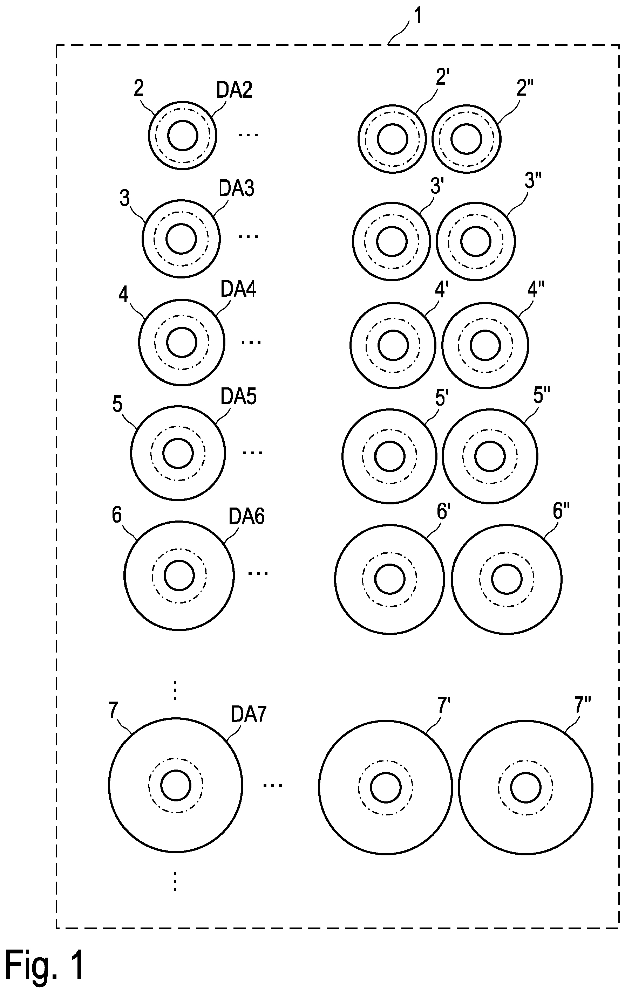

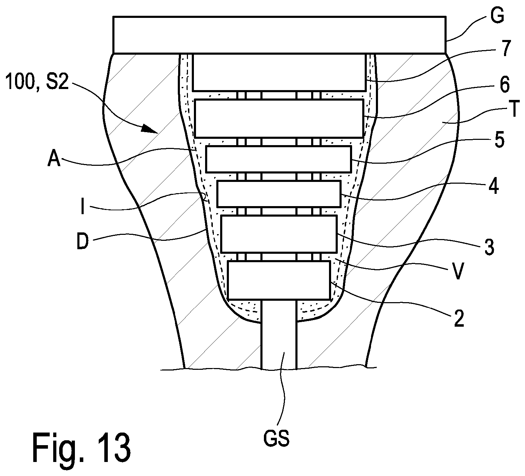

Gemäß Fig. 1 ist ein Knochenimplantatsystem 1 zur Ausbildung eines individualisierten Knochenimplantats 100 (Fig. 13) vorgesehen. Bei der gezeigten Ausführungsform ist das Knochenimplantat 100 zur Verwendung bei einer Kniegelenkersatzoperation vorgesehen und anhand Fig. 13 exemplarisch in einem implantierten Zustand gezeigt. In diesem implantierten Zustand ist das Knochenimplantat 100 proximal in eine Tibia T implantiert. Dabei erfüllt das Knochenimplantat 100 bei der vorliegend gezeigten Verwendung vornehmlich zwei Funktionen. Zum einen füllt das Knochenimplantat 100 einen metaphysären Knochendefekt D im Bereich der proximalen Tibia T auf. Zum anderen stabilisiert das Knochenimplantat 100 ein tibiales Gelenkersatzimplantat G. Bei dem Gelenkersatzimplantat G handelt es sich vorliegend um ein sogenanntes Tibiaplateau, das auf eine dem Fachmann grundsätzlich bekannte Weise proximal an der Tibia T verankert und zum Zusammenwirken mit weiteren Komponenten einer Kniegelenkersatzprothese eingerichtet ist. Um optimale Behandlungsergebnisse zu erzielen, ist es wünschenswert, dass eine Außenkontur A des Knochenimplantats 100 geometrisch auf eine Innenkontur I des Knochendefekts D abgestimmt ist. Die Außenkontur A ist anhand Fig. 13 stark vereinfacht strichliert eingezeichnet und in etwa parallel versetzt zu der Innenkontur I.

Naturgemäß können metaphysäre Knochendefekte unterschiedlichste Geometrien aufweisen. Um dennoch eine möglichst gute Anpassung an die jeweilige Innenkontur zu ermöglichen, sind im Stand der Technik individualisierte Knochenimplantate bekannt. Diese werden nach vorheriger Erfassung der Geometrie des Knochendefekts individualisiert als sog. "Monoblock" einstückig gefertigt. Solche Sonderanfertigungen können zeit- und kostenintensiv sein. Zudem sind speziell für die vorliegende Verwendung als Tibia-Cone bezeichnete Knochenimplantate in unterschiedlichen Größen und Formen am Markt erhältlich. Dabei soll durch eine entsprechende Auswahl der Größe und/oder Form des Knochenimplantats eine verbesserte Anpassung an die jeweilige Innenkontur des Knochendefekts erreicht werden. Dies gelingt jedoch nicht in jedem Fall.

Das Knochenimplantatsystem 1 überwindet die mit dem Stand der Technik einhergehenden Nachteile und gestattet insbesondere eine zeit- und kostensparende Herstellung individualisierter, auf eine vorhandene Defektgeometrie abgestimmter Knochenimplantate. Das Knochenimplantatsystem 1 eignet sich bevorzugt für die vorbeschriebene Verwendung im Rahmen einer Kniegelenkersatzoperation. Dessen ungeachtet können die mittels des Knochenimplantatsystems 1 herstellbaren Knochenimplantate auch im Rahmen sonstiger Gelenkersatzoperationen eingesetzt werden, so beispielsweise im Hüft-, Schulter- und/oder Sprunggelenksbereich.

Das Knochenimplantatsystem 1 weist mehrere unterschiedliche Scheibenelemente 2 bis 7 mit unterschiedlichen Außendurchmessern DA2 bis DA7 auf (Fig. 1).

Anhand Fig. 1 sind exemplarisch sechs im Hinblick auf ihren Außendurchmesser unterschiedliche Scheibenelemente gezeigt. Es versteht sich, dass das Knochenimplantatsystem weniger als die gezeigten sechs, beispielsweise zwei, drei, vier, fünf, oder mehr als die gezeigten sechs, beispielsweise sieben, acht, neun, zehn, unterschiedliche Scheibenelemente aufweisen kann. Dies ist in Fig. 1 anhand der zwischen den Scheibenelementen 6 und 7 sowie unterhalb des Scheibenelements 7 eingezeichneten Punkte symbolisiert.

Die anhand Fig. 1 ersichtlichen Größenverhältnisse zwischen den unterschiedlichen Außendurchmessern DA2 bis DA7 sind als rein exemplarisch zu verstehen. Es versteht sich, dass die unterschiedlichen Außendurchmesser bei zeichnerisch nicht dargestellten Ausführungsformen feiner oder gröber abgestuft sein können.

Zudem weist das Knochenimplantatsystem 1 bei der gezeigten Ausführungsform je Außendurchmesser DA2 bis DA7 mehrere identische Scheibenelemente auf. Insoweit kann auch von einem ersten Satz Scheibenelemente 2, 2', 2" mit dem Außendurchmesser DA2, einem zweiten Satz Scheibenelemente 3, 3', 3" mit dem Außendurchmesser DA3, einem dritten Satz Scheibenelemente 4, 4', 4" mit dem Außendurchmesser DA4 usw. gesprochen werden. Dabei zeigt Fig. 1 exemplarisch jeweils drei Scheibenelemente je Satz. Bei einer zeichnerisch nicht dargestellten Ausführungsform ist lediglich ein einziges Scheibenelement je Außendurchmesser vorgesehen. Bei weiteren nicht gezeigten Ausführungsformen sind beispielsweise zwei, vier, fünf, sechs, oder mehr Scheibenelemente je Satz vorgesehen. Vor diesem Hintergrund können die Scheibenelemente 2, 2', 2" jeweils auch als erstes Scheibenelement bezeichnet werden. Die Scheibenelemente 3, 3', 3" können dementsprechend jeweils als zweites Scheibenelement bezeichnet werden. Die Scheibenelemente 4, 4', 4" können jeweils als drittes Scheibenelement bezeichnet werden. Die Benennung der weiteren Scheibenelemente des Knochenimplantatsystems 1 ergibt sich dementsprechend. Der gebotenen Kürze wegen wird nachfolgend lediglich von dem ersten Scheibenelement 2, dem zweiten Scheibenelement 3, dem dritten Scheibenelement 4, dem vierten Scheibenelement 5 usw. gesprochen.

Die unterschiedlichen Außendurchbesser DA2 bis DA7 sind bei der gezeigten Ausführungsform lediglich im Hinblick auf ihre Abmessungen unterschiedlich. Es versteht sich, dass die unterschiedlichen Außendurchmesser DA2 bis DA7 alternativ oder zusätzlich im Hinblick auf ihre Formgebung unterschiedlich sein können. Insoweit ist die anhand der vorliegenden Figuren gezeigte kreisförmige bzw. kreisringförmige Gestaltung der Außendurchmesser als rein exemplarisch zu verstehen und der vereinfachten zeichnerischen Darstellbarkeit geschuldet. Anstelle der zeichnerisch dargestellten Formgebung können die unterschiedlichen Außendurchmesser DA2 bis DA7 insbesondere elliptisch und/oder im weitesten Sinne abgerundet sein. Durch eine beispielsweises elliptische Formgebung können auch asymmetrische Knochendefekte versorgt werden.

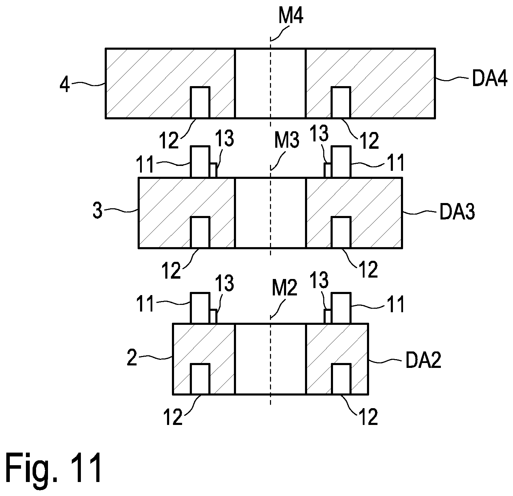

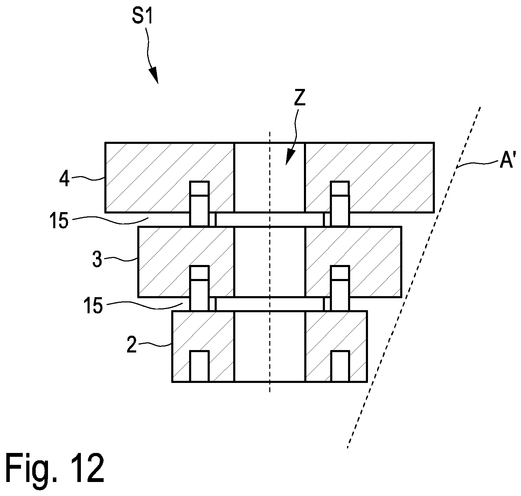

Die unterschiedlichen Scheibenelemente 2 bis 7 sind unter Ausbildung unterschiedlicher Stapelanordnungen unterschiedlich kombiniert axial übereinanderstapelbar. Eine exemplarische erste Stapelanordnung S1 ist anhand der Fig. 11 und 12 gezeigt. Die Stapelanordnung S1 ist beispielhaft durch jeweils eines der ersten, zweiten und dritten Scheibenelemente bzw. durch das erste Scheibenelement 2, das zweite Scheibenelement 3 und das dritte Scheibenelement 4 gebildet. Aufgrund der unterschiedlichen Außendurchmesser DA2, DA3, DA4 und der im Speziellen gewählten Abfolge der Scheibenelemente 2, 3, 4 in axialer Richtung weist die Stapelanordnung S1 eine Außenkontur A' auf. Diese ist anhand Fig. 12 schematisch stark vereinfacht eingezeichnet und im weitesten Sinne konisch. Hierzu im Unterschied ist das Knochenimplantat 100 durch eine unterschiedliche, weitere Stapelanordnung S2 noch näher bezeichneter Scheibenelemente des Knochenimplantatsystems 1 gebildet und weist die bereits erwähnte Außenkontur A auf.

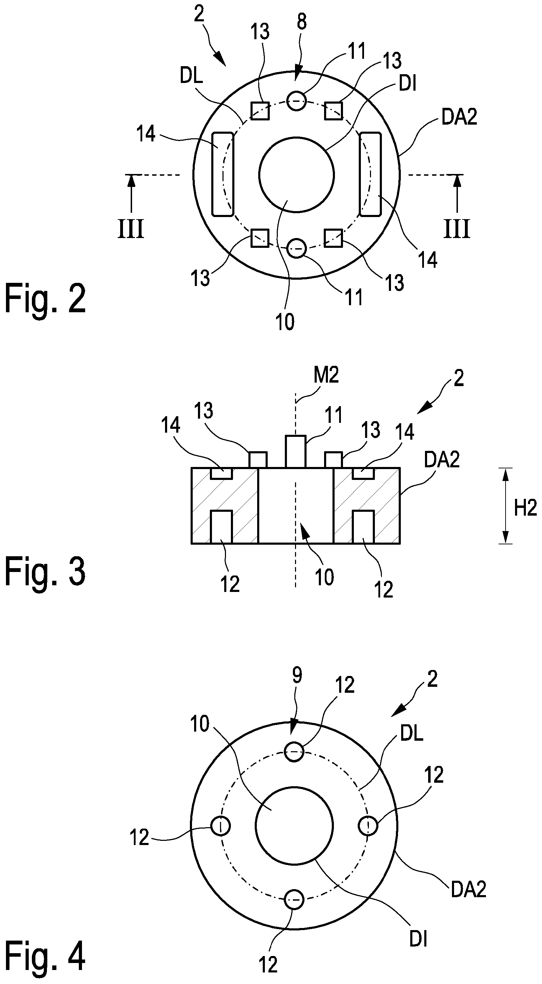

Je nach Auswahl und Stapelreihenfolge der unterschiedlichen Scheibenelemente 2 bis 7 des Knochenimplantatsystems 1 lassen sich unterschiedlichste Stapelanordnungen und damit Knochenimplantate mit unterschiedlichsten Außenkonturen ausbilden. Dabei werden die betreffenden Scheibenelemente nicht lediglich lose aufeinandergelegt, sondern in Radialrichtung und Umfangsrichtung formschlüssig aneinander festgelegt. Zu diesem Zweck weisen die unterschiedlichen Scheibenelemente 2 bis 7 jeweils einen Formschlussabschnitt 8 und einen komplementären Formschlussabschnitt 9 auf (vgl. insb. Fig. 2 bis 10). Die Gestaltung und Funktionsweise der Formschlussabschnitte 8 und der komplementären Formschlussabschnitte 9 sowie die übrige Gestaltung und Funktionsweise der unterschiedlichen Scheibenelemente 2 bis 7 wird nachfolgend anhand der Fig. 2 bis 10 exemplarisch im Hinblick auf das erste Scheibenelement 2, das zweite Scheibenelement 3 und das dritte Scheibenelement 4 erläutert.

Das erste Scheibenelement 2 (Fig. 2 bis 4) ist bei der gezeigten Ausführungsform kreisringförmig gestaltet und kann insoweit auch als Kreisringelement bezeichnet werden. Aufgrund der ringförmigen Gestaltung weist das Scheibenelement 2 einen Innendurchmesser DI auf. Der Innendurchmesser DI ist durch eine Zentralbohrung 10 ausgebildet, die koaxial zu einer Mittellängsachse M2 des ersten Scheibenelements 2 erstreckt ist. Der (erste) Außendurchmesser DA2 ist vorliegend konzentrisch zu dem Innendurchmesser DI. Das erste Scheibenelement 2 weist eine axiale Dicke H2 auf. Bei einer zeichnerisch nicht dargestellten Ausführungsform sind die Scheibenelemente ellipsenförmig gestaltet.

Der Formschlussabschnitt 8 ist an einer Oberseite (Fig. 2) des ersten Scheibenelements 2 angeordnet. Der komplementäre Formschlussabschnitt 9 ist an einer in Axialrichtung gegenüberliegenden Unterseite angeordnet (Fig. 4).

Bei der gezeigten Ausführungsform weist der Formschlussabschnitt 8 zwei um 180° um die Mittellängsachse M2 versetzt angeordnete Verbindungsstifte 11 auf. Die Verbindungsstifte 11 ragen axial von der Oberseite auf und weisen einen kreiszylindrischen Vollquerschnitt auf.

Bei einer zeichnerisch nicht dargestellten Ausführungsform kann der Formschlussabschnitt lediglich einen Verbindungsstift oder mehr als die vorliegend gezeigten zwei Verbindungsstifte aufweisen.

Die Verbindungsstifte 11 können einstückig an der Oberseite des ersten Scheibenelements 2 ausgebildet oder als separate Bauteile gefertigt und mit der Oberseite zusammengefügt sein. Bei der gezeigten Ausführungsform sind die Verbindungsstifte separate Bauteile und jeweils in nicht näher bezeichnete, oberseitig angeordnete Aufnahmebohrungen des ersten Scheibenelements 2 eingesteckt.Der komplementäre Formschlussabschnitt 9 weist vorliegend vier um jeweils 90° um die Mittellängsachse M2 versetzt angeordnete Aufnahmebohrungen 12 auf. Die Aufnahmebohrungen 12 sind parallel zu der Mittellängsachse M2 längserstreckt. Entsprechendes gilt im Übrigen für die Verbindungsstifte 11.

Bei einer zeichnerisch nicht dargestellten Ausführungsform weist der komplementäre Formschlussabschnitt 9 weniger oder mehr als die vorliegend gezeigten vier Aufnahmebohrungen 12 auf.

Die Verbindungsstifte 12 sind auf einem Lochkreis mit einem Lochkreisdurchmesser DL angeordnet. Entsprechendes gilt für die Verbindungsstifte 11. Der Lochkreisdurchmesser DL bzw. der Lochkreis ist anhand der Fig. 2 und 4 strichpunktiert dargestellt.

Bei der gezeigten Ausführungsform ist der Lochkreis in Bezug auf die Mittellängsachse M2 konzentrisch. Konzentrizität liegt vorliegend somit auch in Bezug auf den Innendurchmesser DI und den ersten Außendurchmesser DA2 vor.

Weiter weist das erste Scheibenelement 2 vorliegend wenigstens einen Abstandsabschnitt 13 auf, der in Axialrichtung von der Oberseite abragt. Vorliegend sind insgesamt vier Abstandsabschnitte 13 vorgesehen und jeweils paarweise benachbart zu den Verbindungsstiften 11 im Bereich des Lochkreisdurchmessers DL angeordnet. Die Abstandsabschnitte 13 weisen bei der gezeigten Ausführungsform einen quadratischen Querschnitt auf, was als rein exemplarisch zu verstehen ist. Die Abstandsabschnitte 13 können einstückig an der Oberseite ausgebildet oder als separate Bauteile mit der Oberseite des ersten Scheibenelements 2 verbunden sein. Bei der gezeigten Ausführungsform sind die Abstandsabschnitte 13 jeweils als separate Abstandsstifte gestaltet und in nicht näher ersichtliche, in die Oberseite des ersten Scheibenelements 2 eingebrachte Bohrungen eingesteckt.

Bei einer zeichnerisch nicht gezeigten Ausführungsform sind die Abstandsabschnitte an der Unterseite angeordnet. Zudem versteht sich, dass die Abstandsabschnitte nicht zwingend im Bereich der Lochkreisdurchmessers DL angeordnet sein müssen.

Weiter weist das erste Scheibenelement 2 vorliegend zwei in die Oberseite axial eingesenkte Aufnahmeaussparungen 14 auf. Die Aufnahmeaussparungen 14 können auch als Aufnahmetaschen bezeichnet werden. Die beiden Aufnahmeaussparungen 14 sind in Bezug auf die Mittellängsachse M2 um 180° zueinander versetzt angeordnet und weisen jeweils eine rechteckige Grundform auf. Sowohl die Anordnung als auch die Gestaltung der Aufnahmeaussparungen ist als rein exemplarisch zu erachten.

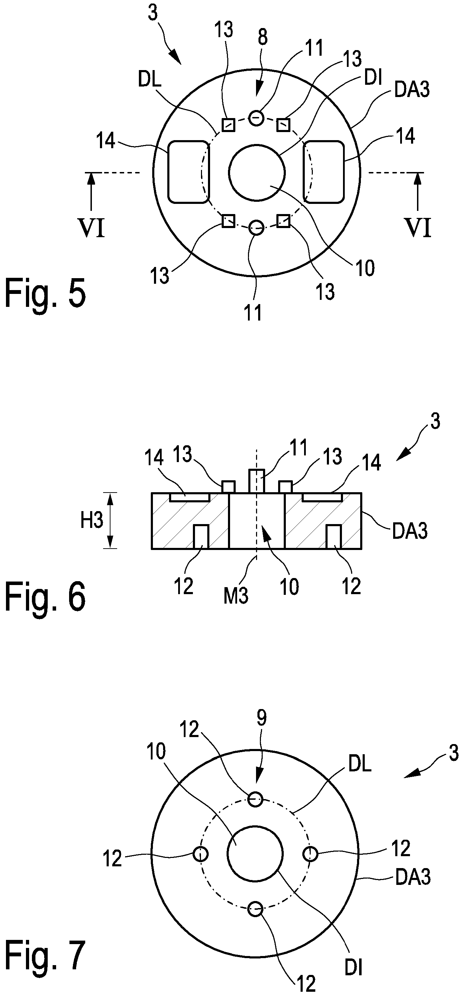

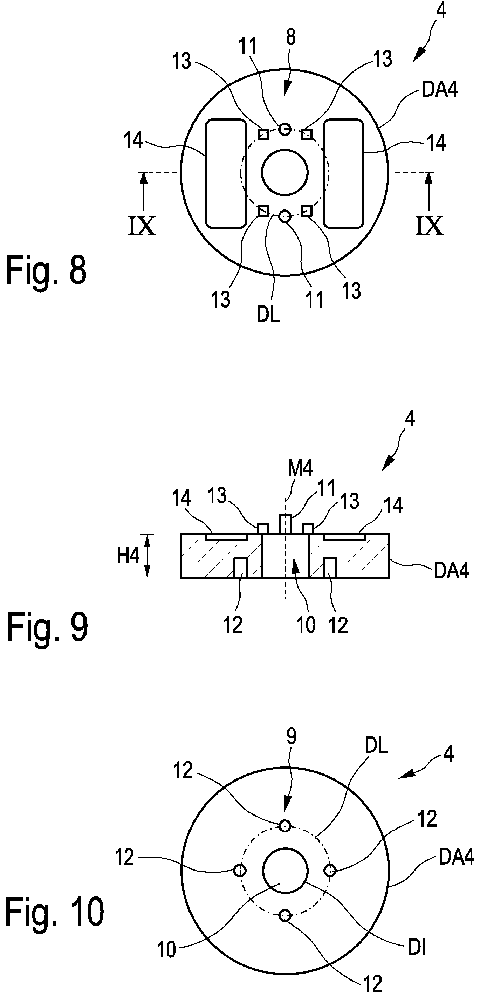

Das zweite Scheibenelement 3 (Fig. 5 bis 7) und das dritte Scheibenelement 4 (Fig. 8 bis 10) sind im Hinblick auf die Gestaltung und/oder Anordnung des Formschlussabschnitts 8 und des komplementären Formschlussabschnitts 9 identisch zu dem ersten Scheibenelement 2. Identische Bauteile und/oder Abschnitte sind dementsprechend mit identischen Bezugszeichenziffern belegt. Das im Hinblick auf den Formschlussabschnitt 8 und den komplementären Formschlussabschnitt 9 im Zusammenhang mit dem ersten Scheibenelement 2 Gesagte gilt sinngemäß entsprechend für das zweite Scheibenelement 3 und das dritte Scheibenelement 4 und im Übrigen für alle weiteren Scheibenelemente des Knochenimplantatsystems 1. Durch die identische Gestaltung und/oder Anordnung der Formschlussabschnitte und der komplementären Formschlussabschnitte ist - vereinfacht ausgedrückt - gewährleistet, dass sämtliche Scheibenelemente des Knochenimplantatsystems 1 unabhängig von der jeweiligen Stapelanordnung aneinander festlegbar sind.

Weiterhin identisch ist die Gestaltung und Anordnung der Abstandsabschnitte 13. Dies ist vorteilhaft, aber nicht zwingend. Bei einer nicht gezeigten Ausführungsform können die Abstandsabschnitte der unterschiedlichen Scheibenelemente 2 bis 7 unterschiedlich gestaltet und/oder angeordnet sein.

Weiter in Übereinstimmung mit dem ersten Scheibenelement 2 weist auch das zweite Scheibenelement 3 in die Oberseite eingesenkte Aufnahmeaussparungen 14 auf. Die Aufnahmeaussparungen 14 des zweiten Scheibenelements 3 unterscheiden sich lediglich im Hinblick auf ihre Größe gegenüber den Aufnahmeaussparungen 14 des ersten Scheibenelements 2, so dass auf ein gesondertes Bezugszeichen verzichtet wird. Entsprechendes gilt sinngemäß im Hinblick auf das dritte Scheibenelement 4 und dessen Aufnahmeaussparungen 14.

Im Übrigen weist das zweite Scheibenelement 3 eine axiale Dicke H3 auf. Das dritte Scheibenelement 4 weist eine axiale Dicke H4 auf. Bei der gezeigten Ausführungsform sind das erste, zweite und dritte Scheibenelement gleich dick, so dass die Dicken H2, H3 und H4 übereinstimmen. Bei einer zeichnerisch nicht dargestellten Ausführungsform weisen unterschiedliche Scheibenelemente unterschiedliche Dicken auf. Zudem ist denkbar, dass Scheibenelemente mit ein- und demselben Außendurchmesser unterschiedliche Dicken aufweisen. So beispielsweise innerhalb des ersten Satzes Scheibenelemente 2, 2', 2" und/oder der übrigen Scheibenelementsätze des Knochenimplantatsystems 1.

Zum Ausbilden der anhand Fig. 12 gezeigten Stapelanordnung S1 wird das erste Scheibenelement 2 bzw. eines der ersten Scheibenelemente 2, 2', 2" ausgewählt. Zudem werden das zweite Scheibenelement 3 und das dritte Scheibenelement 4 bzw. eines der betreffenden Scheibenelemente ausgewählt. Das zweite Scheibenelement 3 wird in axialer Richtung fluchtend zu dem ersten Scheibenelement 2 ausgerichtet, so dass die Mittellängsachsen M2, M3 koaxial ausgerichtet sind. Zudem wird das zweite Scheibenelement 3 in Umfangsrichtung derart relativ zu dem ersten Scheibenelement 2 positioniert, dass die Verbindungsstifte 11 des letzteren in die Aufnahmebohrungen 12 des zweiten Scheibenelements 3 eingreifen können. Hiernach wird das zweite Scheibenelement 3 in axialer Richtung auf das erste Scheibenelement 2 aufgesteckt. Hierdurch wird sowohl radial als auch in Umfangsrichtung ein Formschluss zwischen den Verbindungsstiften 11 und den Aufnahmebohrungen 12 ausgebildet. Aufgrund der oberseitig an dem ersten Scheibenelement 2 angeordneten Abstandsabschnitte 13 ergibt sich ein in axialer Richtung erstreckter Radialspalt 15 (Fig. 12). Hiernach wird das dritte Scheibenelement 4 unter koaxialer Ausrichtung der Mittellängsachsen M3, M4 auf das zweite Scheibenelement 3 gestapelt. Hierdurch wird wiederum ein entsprechender Formschluss zwischen den Verbindungsstiften 11 des zweiten Scheibenelements 3 und den Aufnahmebohrungen 12 des dritten Scheibenelements 4 ausgebildet. Zudem ergibt sich auch zwischen dem zweiten Scheibenelement 3 und dem dritten Scheibenelement 4 ein Radialspalt 15.

Im Hinblick auf die Fig. 11 und 12 ist erwähnenswert, dass sowohl die Verbindungsstifte 11 als auch die Abstandsabschnitte 13 des dritten Scheibenelements 4 aufgrund ihrer jeweiligen Gestaltung als separate Bauteile entfernt werden können. Dies ist vorteilhaft, aber nicht zwingend.

In der Stapelanordnung S1 bilden die Zentralbohrungen 10 bzw. die Innendurchmesser DI eine zylindrische Aufnahmeaussparung Z aus. Die Aufnahmeaussparung Z ist zur Aufnahme eines Schaftabschnitts GS des Gelenkersatzimplantats G geeignet (Fig. 13).

Die zwischen den Scheibenelementen 2, 3, 4 ausgebildeten Axialspalte 15 ermöglichen das Eindringen und/oder Einbringen einer Verbindungsmasse V (vgl. Fig. 13). Hierdurch kann die Stapelanordnung S1 zusätzlich stabilisiert werden. Die Verbindungsmasse V kann alternativ oder zusätzlich vor dem Aufeinanderstapeln der Scheibenelemente 2, 3, 4 in deren Aufnahmeaussparungen 14 eingebracht werden.

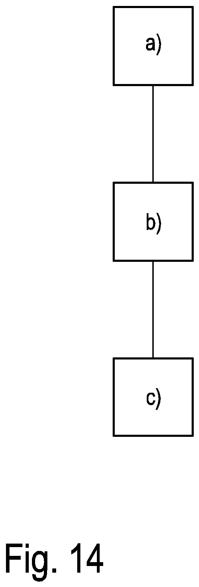

Anhand Fig. 14 ist eine Ausführungsform eines erfindungsgemäßen Verfahrens zur Herstellung des Knochenimplantats 100 unter Verwendung des Knochenimplantatsystems 1 schematisch verdeutlicht. Das Verfahren weist die Schritte a), b) und c) auf.

In dem Schritt a) wird zunächst die Innenkontur I des metaphysären Knochendefekts D messtechnisch erfasst. Hierfür geeignete Messverfahren sind dem Fachmann grundsätzlich bekannt. Beispielsweise kann eine sogenannte Time-of-Flight-Kamera verwendet werden.

Der Schritt b) sieht ein Ermitteln der zum Auffüllen des Knochendefekts D erforderlichen Außenkontur A des Knochenimplantats 100 vor. Dabei wird die Außenkontur A in Abhängigkeit der zuvor erfassten Innenkontur I, vorzugsweise computergestützt und/oder simulationsbasiert, ermittelt.

Der Schritt c) sieht ein Auswählen, Stapeln und Aneinanderfestlegen mehrerer unterschiedlicher Scheibenelemente des Knochenimplantatsystems 1 vor. Dabei werden die mehreren Scheibenelemente in Abhängigkeit der ermittelten Außenkontur A, vorzugsweise intra-operativ, ausgewählt und/oder gestapelt. Das Auswählen erfolgt durch medizinisches Personal und vorzugsweise computergestützt und/oder simulationsbasiert. Vorliegend werden jeweils das bzw. ein erstes Scheibenelement 2, ein zweites Scheibenelement 3, ein drittes Scheibenelement 4, ein viertes Scheibenelement 5, ein fünftes Scheibenelement 6 und ein sechstes Scheibenelement 7 ausgewählt und übereinandergestapelt. Die Verbindung der besagten Scheibenelemente erfolgt mittels der Formschlussabschnitte 8 und der komplementären Formschlussabschnitte 9, wie zuvor beschrieben. Anhand Fig. 13 sind einzelne der Scheibenelemente 2 bis 7 mit unterschiedlicher Dicke eingezeichnet. Hierdurch soll nochmals verdeutlicht werden, dass nicht notwendigerweise sämtliche Scheibenelemente des Knochenimplantatsystems 1 ein- und dieselbe axiale Dicke aufweisen müssen. Nachdem die Scheibenelemente 2 bis 7 manuell aufeinandergestapelt und formschlüssig aneinander festgelegt wurden, kann das Knochenimplantat 100 in den Knochendefekt D eingesetzt und dort mittels der Verbindungsmasse V, die vorliegend ein Knochenzement ist, verankert werden. Die Verbindungmasse V kann bereits vor dem Einsetzen des Knochenimplantats 100 in die zylindrische Aufnahmeaussparung Z oder zwischen die unterschiedlichen Scheibenelemente 2 bis 7 eingebracht werden. Hierdurch wird eine zusätzliche Stabilisierung des Knochenimplantats 100 erreicht. Nach Verankerung des Knochenimplantats 100 kann das Gelenkersatzimplantat G proximal an der Tibia T angebracht werden. Hierbei wird der Schaftabschnitt GS axial in die zylindrische Aufnahmeaussparung Z eingesteckt und in radialer Richtung in dieser verankert.

## Patentansprüche

1. Knochenimplantatsystem (1) zur Herstellung eines individualisierten Knochenimplantats (100), aufweisend
mehrere unterschiedliche Scheibenelemente (2 bis 7) mit unterschiedlichen Außendurchmessern (DA2 bis DA7),
wobei die Scheibenelemente (2 bis 7) unter Ausbildung unterschiedlicher Stapelanordnungen (S1, S2) unterschiedlich kombiniert axial übereinanderstapelbar sind,
wobei die Scheibenelemente (2 bis 7) jeweils einen an einer Oberseite angeordneten Formschlussabschnitt (8) und einen an einer axial gegenüberliegenden Unterseite angeordneten komplementären Formschlussabschnitt (9) aufweisen, mittels derer die Scheibenelemente in den unterschiedlichen Stapelanordnungen (S1, S2) in Radialrichtung und/oder Umfangsrichtung formschlüssig aneinander festlegbar sind,
**dadurch gekennzeichnet, dass** die unterschiedlichen Stapelanordnungen (S1, S2) aufgrund der unterschiedlichen Außendurchmesser (DA2 bis DA7) der Scheibenelemente (2 bis 7) unterschiedliche Außenkonturen (A, A') aufweisen.

2. Knochenimplantatsystem (1) nach Anspruch 1, **dadurch gekennzeichnet, dass** die Scheibenelemente (2 bis 7) jeweils ringförmig gestaltet sind und einen, vorzugsweise identischen, Innendurchmesser (DI) aufweisen.

3. Knochenimplantatsystem (1) nach Anspruch 1 oder 2, **dadurch gekennzeichnet, dass** die Scheibenelemente (2 bis 7) jeweils wenigstens einen in Axialrichtung von der Oberseite oder der Unterseite aufragenden Abstandsabschnitt (13) aufweisen, wodurch die Scheibenelemente (2) bis (7) in den unterschiedlichen Stapelanordnungen (S1, S2) unter Ausbildung von Axialspalten (15) übereinanderstapelbar sind.

4. Knochenimplantatsystem (1) nach einem der vorhergehenden Ansprüche, **dadurch gekennzeichnet, dass** die Scheibenelemente (2 bis 7) jeweils wenigstens eine in die Oberseite oder die Unterseite eingesenkte Aufnahmeaussparung (14) aufweisen, die zur Aufnahme einer Verbindungsmasse (V) vorgesehen ist.

5. Knochenimplantatsystem (1) nach einem der vorhergehenden Ansprüche, **dadurch gekennzeichnet, dass** die Formschlussabschnitte (8) jeweils wenigstens einen axial aufragenden Verbindungsstift (11) aufweisen, und dass die komplementären Formschlussabschnitte (9) jeweils wenigstens eine in Axialrichtung eingebrachte Aufnahmebohrung (12) aufweisen oder umgekehrt.

6. Knochenimplantatsystem (1) nach Anspruch 5, **dadurch gekennzeichnet, dass** die Scheibenelemente (2 bis 7) jeweils mehrere in Umfangsrichtung versetzt angeordnete Verbindungsstifte (11) und/oder Aufnahmebohrungen (12) aufweisen, wobei die Anordnungen der Verbindungsstifte (11) und/oder der Aufnahmebohrungen (12) der unterschiedlichen Scheibenelemente (2 bis 7) ein identisches Bohrbild ausbilden.

7. Knochenimplantatsystem (1) nach einem der vorhergehenden Ansprüche, **dadurch gekennzeichnet, dass** wenigstens drei, bevorzugt mehr als sechs, besonders bevorzugt mehr als zwölf, unterschiedliche Scheibenelemente (2 bis 7) vorgesehen sind.

8. Knochenimplantat (100) hergestellt unter Verwendung eines Knochenimplantatsystems (1) nach einem der Ansprüche 1 bis 7, aufweisend mehrere in Axialrichtung übereinandergestapelte Scheibenelemente (2 bis 7) mit unterschiedlichen Außendurchmessern (DA2 bis DA7), wobei die übereinandergestapelten Scheibenelemente (2 bis 7) mittels oberseitig an den Scheibenelementen (2 bis 7) angeordneter Formschlussabschnitte (8) und unterseitig an den Scheibenelementen (2 bis 7) angeordneter komplementärer Formschlussabschnitte (9) in Radialrichtung und/oder Umfangsrichtung formschlüssig aneinander festgelegt sind.

9. Verfahren zur Herstellung eines individualisierten Knochenimplantats (100) nach Anspruch 8 unter Verwendung eines Knochenimplantatsystems (1) nach einem der Ansprüche 1 bis 7, aufweisend die Schritte:
a) Erfassen einer Innenkontur (I) eines Knochendefekts (D);
b) Ermitteln einer zum Auffüllen des Knochendefekts (D) erforderlichen Außenkontur (A) des Knochenimplantats (100) in Abhängigkeit der erfassten Innenkontur (I);
c) Auswählen, Stapeln und Aneinanderfestlegen mehrerer unterschiedlicher Scheibenelemente (2 bis 7) des Knochenimplantatsystems (1), wobei die mehreren Scheibenelemente (2 bis 7) in Abhängigkeit der ermittelten Außenkontur (A) ausgewählt und/oder gestapelt werden.

## Claims

1. Bone implant system (1) for producing an individualized bone implant (100), having
a plurality of different disc elements (2 to 7) with different external diameters (DA2 to DA7),
wherein the disc elements (2 to 7) are able to be stacked axially one on top of another in different combinations in order to form different stack arrangements (S1, S2),
wherein the disc elements (2 to 7) each have a form-fitting portion (8) arranged on an upper face and a complementary form-fitting portion (9) arranged on an axially opposite lower face, by means of which the disc elements in the different stack arrangements (S1, S2) are able to be interlockingly fastened to one another in radial direction and/or circumferential direction,
**characterized in that** the different stack arrangements (S1, S2) have different outer contours (A, A') on account of the different external diameters (DA2 to DA7) of the disc elements (2 to 7).

2. Bone implant system (1) as claimed in claim 1, **characterized in that** the disc elements (2 to 7) are each ring-shaped and have a, preferably identical, internal diameter (DI).

3. Bone implant system (1) as claimed in claim 1 or 2, **characterized in that** the disc elements (2 to 7) each have at least one spacer portion (13) protruding in the axial direction from the upper face or the lower face, as a result of which the disc elements (2 to 7) are able to be stacked one on top of another in the different stack arrangements (S1, S2) while forming axial gaps (15).

4. Bone implant system (1) as claimed in one of the preceding claims, **characterized in that** the disc elements (2 to 7) each have at least one receiving recess (14) which is sunk into the upper face or the lower face and which is provided for receiving a connecting compound (V).

5. Bone implant system (1) as claimed in one of the preceding claims, **characterized in that** the form-fitting portions (8) each have at least one axially protruding connection pin (11), and **in that** the complementary form-fitting portions (9) each have at least one receiving bore (12) introduced in the axial direction, or vice versa.

6. Bone implant system (1) as claimed in claim 5, **characterized in that** the disc elements (2 to 7) each have a plurality of connection pins (11) and/or receiving bores (12) arranged offset in the circumferential direction, the arrangements of the connection pins (11) and/or of the receiving bores (12) of the different disc elements (2 to 7) forming an identical bore pattern.

7. Bone implant system (1) as claimed in one of the preceding claims, **characterized in that** at least three, preferably more than six, particularly preferably more than twelve, different disc elements (2 to 7) are provided.

8. Bone implant (100) produced using a bone implant system (1) as claimed in one of claims 1 to 7, the bone implant (100) having a plurality of disc elements (2 to 7) stacked one on top of another in the axial direction with different external diameters (DA2 to DA7), the disc elements (2 to 7) stacked one on top of another being interlockingly fastened to one another in radial direction and/or circumferential direction by means of form-fitting portions (8) arranged on the upper face of the disc elements (2 to 7) and complementary form-fitting portions (9) arranged on the lower face of the disc elements (2 to 7).

9. Method for producing an individualized bone implant (100) according to claim 8, comprising the steps of:
a) recording an inner contour (I) of a bone defect (D);
b) determining an outer contour (A) of the bone implant (100), required to fill the bone defect (D), in accordance with the recorded inner contour (I);
c) selecting, stacking and mutually fastening a plurality of different disc elements (2 to 7) of the bone implant system (1), the plurality of disc elements (2 to 7) being selected and/or stacked in accordance with the recorded outer contour (A).

## Revendications

1. Système d'implant osseux (1) pour la fabrication d'un implant osseux individualisé (100), présentant plusieurs éléments en forme de disque différents (2 à 7) ayant des diamètres extérieurs différents (DA2 à DA7),
les éléments en forme de disque (2 à 7) pouvant être empilés axialement les uns sur les autres en réalisant différents agencements d'empilement (S1, S2) combinés différemment,
les éléments en forme de disque (2 à 7) présentant chacun une section à complémentarité de forme (8) agencée sur un côté supérieur et une section à complémentarité de forme (9) complémentaire agencée sur un côté inférieur axialement opposé, au moyen desquelles les éléments en forme de disque peuvent être fixés les uns aux autres par complémentarité de forme dans les différents agencements d'empilage (S1, S2) dans la direction radiale et/ou dans la direction périphérique, **caractérisé en ce que** les différents agencements d'empilement (S1, S2) présentent des contours extérieurs (A, A') différents en raison des différents diamètres extérieurs (DA2 à DA7) des éléments en forme de disque (2 à 7).

2. Système d'implant osseux (1) selon la revendication 1, **caractérisé en ce que** les éléments en forme de disque (2 à 7) sont chacun de forme annulaire et présentent un diamètre intérieur (DI), de préférence identique.

3. Système d'implant osseux (1) selon la revendication 1 ou 2, **caractérisé en ce que** les éléments en forme de disque (2 à 7) présentent chacun au moins une section d'espacement (13) qui fait saillie dans la direction axiale à partir du côté supérieur ou du côté inférieur, moyennant quoi les éléments en forme de disque (2) à (7) peuvent être empilés les uns sur les autres dans les différents agencements d'empilement (S1, S2) en réalisant des fentes axiales (15).

4. Système d'implant osseux (1) selon l'une quelconque des revendications précédentes, **caractérisé en ce que** les éléments en forme de disque (2 à 7) présentent chacun au moins un évidement de réception (14) creusé dans le côté supérieur ou le côté inférieur, prévu pour recevoir une masse de liaison (V).

5. Système d'implant osseux (1) selon l'une quelconque des revendications précédentes, **caractérisé en ce que** les sections à complémentarité de forme (8) présentent chacune au moins une tige de liaison (11) en saillie axiale, et **en ce que** les sections à complémentarité de forme complémentaires (9) présentent chacune au moins un trou de réception (12) pratiqué dans la direction axiale, ou inversement.

6. Système d'implant osseux (1) selon la revendication 5, **caractérisé en ce que** les éléments en forme de disque (2 à 7) présentent chacun plusieurs tiges de liaison (11) et/ou trous de réception (12) agencés de manière décalée dans la direction périphérique, les agencements des tiges de liaison (11) et/ou des trous de réception (12) des différents éléments en forme de disque (2 à 7) réalisant un schéma de perçage identique.

7. Système d'implant osseux (1) selon l'une quelconque des revendications précédentes, **caractérisé en ce qu'**il est prévu au moins trois, de préférence plus de six, de manière particulièrement préférée plus de douze, éléments en forme de disque différents (2 à 7).

8. Implant osseux (100) fabriqué en utilisant un système d'implant osseux (1) selon l'une quelconque des revendications 1 à 7, présentant plusieurs éléments en forme de disque (2 à 7) empilés les uns sur les autres dans la direction axiale avec des diamètres extérieurs différents (DA2 à DA7), les éléments en forme de disque (2 à 7) empilés les uns sur les autres étant fixés les uns aux autres par complémentarité de forme dans la direction radiale et/ou dans la direction périphérique au moyen de sections à complémentarité de forme (8) agencées sur le côté supérieur des éléments en forme de disque (2 à 7) et de sections à complémentarité de forme (9) agencées sur le côté inférieur des éléments en forme de disque (2 à 7).

9. Procédé de fabrication d'un implant osseux individualisé (100) selon la revendication 8, en utilisant un système d'implant osseux (1) selon l'une quelconque des revendications 1 à 7, présentant les étapes suivantes :
a) la détection d'un contour intérieur (I) d'un défaut osseux (D) ;
b) la détermination d'un contour extérieur (A) de l'implant osseux (100) nécessaire au comblement du défaut osseux (D) en fonction du contour intérieur (I) détecté ;
c) la sélection, l'empilement et la fixation les uns aux autres de plusieurs éléments en forme de disque différents (2 à 7) du système d'implant osseux (1), les plusieurs éléments en forme de disque (2 à 7) étant sélectionnés et/ou empilés en fonction du contour extérieur (A) déterminé.
